# EUROPÄISCHE PATENTSCHRIFT

(11) **EP 3 383 200 B1**
(45) Veröffentlichungstag und Bekanntmachung des Hinweises auf die Patenterteilung: **06.11.2019**
(21) Anmeldenummer: 15816677.7
(22) Anmeldetag: 01.12.2015
(51) Int. Cl.: A23L 27/30, A23L 27/00, A23L 27/10

(54) **STOFFGEMISCHE**
COMPOSITIONS
COMPOSITIONS DE MATIÈRE

(43) Veröffentlichungstag der Anmeldung: 10.10.2018
(73) Patentinhaber: Symrise AG, 37603 Holzminden (DE)
(72) Erfinder: KIEFL, Johannes, 37603 Holzminden (DE); PAETZ, Susanne, 37671 Höxter (DE); LEY, Jakob, 37603 Holzminden (DE); KRAMMER, Gerhard, 37603 Holzminden (DE); RIESS, Thomas, 37603 Holzminden (DE); LANGER, Kathrin, 37586 Dassel-Hilwartshausen (DE); KINDEL, Günter, 37671 Höxter (DE); VERWOHLT, Morine, 37627 Linnenkamp (DE); GEISSLER, Torsten, 37574 Einbeck (DE); GROSS, Egon, 37603 Holzminden (DE)
(74) Vertreter: Eisenführ Speiser
(86) Internationale Anmeldenummer: PCT/EP2015/078255
(87) Internationale Veröffentlichungsnummer: WO 2017/092796

(56) Entgegenhaltungen:
- EP-A1- 2 340 719
- EP-A2- 2 193 785
- US-A1- 2015 320 101
- WIBKE S. U. ROLAND ET AL: "Bitter Taste Receptor Activation by Flavonoids and Isoflavonoids: Modeled Structural Requirements for Activation of hTAS2R14 and hTAS2R39", JOURNAL OF AGRICULTURAL AND FOOD CHEMISTRY, Bd. 61, Nr. 44, 6. November 2013 (2013-11-06), Seiten 10454-10466, XP055297209, US ISSN: 0021-8561, DOI: 10.1021/jf403387p

## Beschreibung

### GEBIET DER ERFINDUNG

Die Erfindung befindet sich auf dem Gebiet der Nahrungsmittel, speziell der Aromastoffe mit süßoptimierenden Eigenschaften und betrifft Zubereitungen, die Phloretin und einen weiteren Aromastoff enthalten, der die unerwünschten geschmacklichen Nebennoten dieses Stoffes ausgleicht. Der Gegenstand der Erfindung ist durch die anhängigen Ansprüche definiert.

### STAND DER TECHNIK

In der Lebensmittelindustrie werden vermehrt Produkte entwickelt, die dem Verbraucher die Aufnahme wertvoller Komponenten wie z.B. Flavonoide zur Unterstützung der Gesunderhaltung ermöglichen. Diese Produkte enthalten meist einen verringerten Gehalt an Zucker um eine Kalorienreduktion des Lebensmittels zu erreichen. Die Zuckerreduktion geht dabei jedoch mit einem Verlust des Süßeindrucks und des Körpers sowie der Mundfülle des Lebensmittels einher. Daher werden oft Stoffe wie Phloretin eingesetzt um den fehlenden Süßgeschmacks auszugleichen. Dabei treten jedoch Nebennoten wie unerwünschte Bitterkeit, Adstringenz und/oder fehlende Mundfülle auf.

Aus dem Stand der Technik sind Produkte, die Dihydrochalkone wie Phloretin zur Süßoptimierung nthalten, bekannt (z.B. EP 2868209 A1 (SYMRISE). Ebenfalls bekannt sind Verfahren zur Herstellung dieser Stoffe durch extraktive, biotechnologische sowie chemische Prozesse (EP 2742983 A1 (SYMRISE); EP 2692729 A1 (SYMRISE), CN 103230408 A1 (**FOS-HAN**). Zudem existieren Unternehmungen, den Gehalt von Phloretin in Extrakten durch Anreicherungsverfahren zu erhöhen (CN 104292094 (GUILIN).

Mischungen bestehend aus Phloretin und anderen Flavonoiden wie Naringenin werden zum Bespiel zur positiven Beeinflussung der Flavonoidaufnahme im Darm verwendet (WO0234073). Bekannt sind auch Verfahren zur Extraktion von Naringenin aus Citrusfrüchten wie z.B. Pampelmusen (CN 104277024 (GUILIN).

Weiterhin werden Mischungen von Flavonoiden mit Phloretin zur Maskierung des bitteren Nachgeschmacks von Süßstoffen wie Steviolgylcosiden beschrieben (EP 2220945 A1). Hierbei handelt es sich jedoch bei der Süßkomponente mit bitterem Nachgeschmack nicht um Phloretin sondern um andere Süßstoffe wie Thaumatin, Neohesperedin dihydrochalkon und dergleichen. Ferner wird Phloretin in diesem Fall zur Maskierung anderer Stoffe eingesetzt und nicht selbst maskiert.

Hydroxyflavone sind ebenfalls zur Maskierung des bitteren Geschmacks beschrieben (EP 1258200 A1)**.** Aus der WO 2015 169769 A1 (FIRMNENICH) sind gesüßte Getränke bekannt, denen man zur Steigerung der Süßwirkung 30 bis 200 ppm Naringenin zusetzt. In der EP 2923584 A1 (IFF) wird vorgeschlagen, die Süße eines süßen Geschmacksmodulators durch Zugabe von Naringenin zu steigern. Die Kombination aus Naringenin und Phloretin wird in diesen Schriften nicht ausdrücklich genannt. Zudem bietet die unten beschriebene Naringenin den zusätzlichen Vorteil einer Abrundung des Geschmackprofils sowie einer zuckerähnlicheren Geschmacks gegenüber der Verwendung von Phloretin. In US2015/0320101 A1 wird die Verwendung von Naringenin zur Modulierung des Bittergeschmacks von Süßstoffen beschrieben.

Trotz der süßoptimierenden Eigenschaften von Phloretin weisen zuckerreduzierte Zubereitungen, die Phloretin enthalten einen bitteren, adstringierenden Nachgeschmack oder Fehlgeschmack (Off-Taste) auf.

Die Wirkung einer Substanz zur Süßoptimierung ist dadurch gekennzeichnet, dass sie in Kombination mit mindestens einem weiteren süßen Stoff, der in einer Konzentration vorliegt, die für sich schon eine deutlich wahrnehmbare Süßintensität verursacht, eine erhöhte Sofortsüße, eine erhöhte Süßintensität, eine erhöhte Mundfülle und dabei eine geringere Bitterkeit und geringere Adstringenz hervorruft.

Die Aufgabe der vorliegenden Erfindung hat daher zum einen darin bestanden, das geschmacklich nicht einwandfreie sensorische Profil sowohl von Phloretin abzurunden und den bitteren Nachgeschmack zu reduzieren und zum anderen Stoffgemische und Aromazubereitungen zur Verfügung zu stellen, die sowohl über ein hohes Süßoptimierungspotential als auch über ein abgerundetes Geschmacksprofil verfügen.

### BESCHREIBUNG DER ERFINDUNG

Ein erster Gegenstand der Erfindung betrifft Stoffgemische, enthaltend
(a) Phloretin,
(b) Naringenin,
(c) mindestens einem weiteren süßen Stoff, der von Phloretin und Naringenin verschieden ist sowie gegebenenfalls
(d) mindestens einen Aromastoff, wobei die Komponenten (a) und (b) im Gewichtsverhältnis von 99:1 bis 1:99 enthalten sind.

Überraschenderweise wurde gefunden, dass die Mischungen über eine optimierte Süßungswirkung verfügen und in besonderer Weise geeignet sind, das Profil des süßen Geschmacks von Nahrungsmitteln abzurunden und zu optimieren. Insbesondere wird in der Kombination mit Naringenin und dem Phloretin der bittere bis stechende Nachgeschmack von süßen Stoffen maskiert und gleichzeitig die Sofortsüße und die Süßintensität gesteigert. Die Stoffgemische lassen sich durch Zusatz weiterer Aromastoffe auch zu Aromazusammensetzungen erweitern. Beide, sowohl die Stoffgemische als auch die Aromazusammensetzung, sind ideal geeignet, um in Nahrungsmittel eingearbeitet zu werden.

### PHLORETIN

Phloretin wird vor allem aus Apfelgewächsen (*Malus sp*.) durch Extraktion gewonnen, wobei auch biotechnologische Methoden zur Herstellung bekannt sind (EP2692729). In Pflanzen liegt Phloretin (Formel I) in Form des 2'-β-Glucosids Phloridzin vor und kann als solches extrahiert und nachfolgend oder in einem Schritt gespalten werden. Phloretin ist als Dihydrochalkon der Klasse der Polyketide zuzuordnen und nah verwandt mit den Flavonoiden.

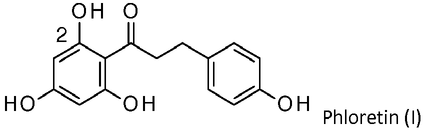

Phloretin hat selbst nur eine schwache Süßwirkung kann allerdings den Süßgeschmackseindruck von anderen süßen Stoffen verstärken, wie in EP 1,998,636-B1 beschrieben. Allerdings zeigt es neben der ebenfalls bekannten bittermaskierenden Eigenschaft (vgl. EP 1,972,203-B1) gegen weitere Bitterstoffe eine Eigenbitterkeit, insbesondere bei höheren Konzentrationen, z.B. bei 50 ppm. Der Stoff selbst lässt in an sich bekannter Weise durch Extraktion der Blätter, der Zweige, der Wurzelrinde oder der Früchte von *Malus sp.* z.B. mit wässrigem Ethanol und anschließender Hydrolyse z.B. mit Enzymen, sauren Ionentauschern, Mineral- und/oder organischen Säuren herstellen, wie z.B. in EP 2,017,272-B1 beschrieben, und durch anschließender Aufreinigung durch z.B. chromatographische Verfahren gewinnen.

### NARINGENIN

Naringenin (Formel II) wird hauptsächlich in Form seiner Glycoside, z.B. Naringin, Narirutin und Prunin, aus Zitrusfrüchten der Gattung *Citrus sp.* vor allem aus Grapefruit (*Citrus paradisi*), Bitterorange (*Citrus* × *aurantium* L.) oder Bergamotte (*Citrus bergamia*), Zitrone (Citrus medica, Citrus limon), Orange (*Citrus sinensis*) gewonnen und besitzt als solches keine auffälligen Geschmackseigenschaften. Durch die enzymatische Reaktion wird in Pflanzen hauptsächlich S-Enantiomer gebildet. Bei der extraktiven Gewinnung von Naringin kann das chirale Zentrum des Aglycons vollständig oder wenigstens zum Teil racemisiert werden, so dass man bei einer nachfolgenden Glycosidspaltung partiell oder vollständig racemisches Naringenin erhält.

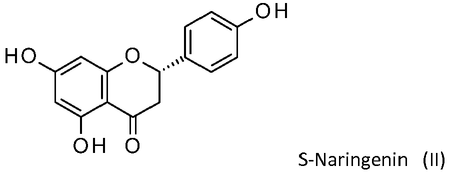

Der Gehalt an Naringenin bzw. Naringin unterscheidet sich in verschiedenen Citrus-Spezies und variiert nach Anbaugebiet, Pflanzensorte, Pflanzenteil und Reifezustand. Naringenin wird z.B. freigesetzt bei der Saftproduktion oder Lagerung, so dass ein geringer Anteil in verzehrsfertigen Produkten zu finden ist. Naringenin in freier Form kommen nicht in einer für die erfinderische Wirkung ausreichend hohen Konzentration (> 5 ppm) in Orangen-, Zitronen oder Grapefruitsäften vor. So wurde in Yanez, J. A.; Remsberg, C. M.; Miranda, N. D.; Vega-Villa, K. R.; Andrews, P. K.; Davies, N. M., Pharmacokinetics of selected chiral flavonoids: hesperetin, naringenin and eriodictyol in rats and their content in fruit juices. Biopharmaceutics & Drug Disposition 2008, 29, (2), 63-82, Naringenin in Orangensäften mit maximal 0,5 ppm, in Grapefruitsäften bis ca. 2,06 ppm gefunden, was aber eine regelmäßig zu geringe Konzentration zur Entfaltung der süßoptimierenden Wirkung ist, insbesondere da in den gleichen Säften die 250fache Konzentration des sehr bitteren Naringins zu finden ist.

Bei Naringenin, das im Sinne der vorliegenden Erfindung bevorzugt ist, kann es sich sowohl als R-Naringenin als auch um S-Naringenin sowie Mischungen der beiden Enantiomere handeln. Naringenin, im Sinne der vorliegenden Erfindung, liegt bevorzugt als Gemisch von R- und S-Naringenin vor wobei etwa 0,01 bis etwa 99,9, vorzugsweise etwa 50 bis 99,5, besonders bevorzugt 90 bis 99 Gew.-%, S-Naringenin in Bezug auf die Gesamtmenge an Naringenin vorliegen können.

Diese Mischungen lassen sich nach dem weiter oben beschriebenen enzymatischen oder fermentativen Verfahren der EP 2692729 A1 aus einem oder mehreren der folgenden Flavonoide erhalten: Naringenin, Naringin, Narirutin. Soweit diese Schriften die Herstellung der Mischungen beschreiben, wird ihr Inhalt hiermit durch Bezugnahme eingeschlossen.

### SÜSSE STOFFE

Als süße Stoffe oder süß schmeckende Stoffe kommen zunächst süß schmeckende Kohlenhydrate und speziell Zucker (Saccharose) in Frage, wie etwa Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrine verschiedener Polymerisationsgrade. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Honig, Ahornsirup (Acer ssp.) oder Agaven (Agavendicksaft).

In Betracht kommen auch
(i) synthetische, d.h. in der Regel enzymatisch hergestellte Stärke oder Zuckerhydrolysate (Invertzucker, Fructosesirup);
(ii) Fruchtkonzentrate (z.B. auf Basis von Äpfeln oder Birnen);
(iii) Zuckeralkohole (z.B. Erythritol, Threitol, Arabitol, Ribotol, Xylitol, Sorbitol, Mannitol, Dulcitol, Lactitol);
(iv) Süß schmeckende Proteine und Peptide (z.B. Miraculin, Monellin, Thaumatin, Curculin, Brazzein);
(v) Süßstoffe (z.B. Magap, Natriumcyclamat, Acesulfam K, Neohesperidindihydrochalkon, Saccharin Natriumsalz, Aspartam, Superaspartam, Neotam, Alitam, Sucralose, Stevioside, Rebaudioside, Rubusoside, Suavioside, Mogroside, Lugduname, Carrelame, Sucrononate, Sucrooctate, Monatin, Phenylodulcin, Phyllodulcin, Abrusoside (z.B. in WO 2015 091,298 beschrieben);
(vi) natürlich vorkommende Süßstoffe, ausgewählt aus der Untergruppe bestehend aus Steviosid, Steviolbiosid, Rebaudiosid A, weiteren Steviolglycosiden wie Rebaudiosid B, Rebaudiosid C, Rebaudiosid D, Rebaudiosid E, Rebaudiosid F, Rebaudiosid G, Rebaudiosid H, Dulcosid und/oder Rubusosid, Oslandin, Polypodosid A, Strogin 1, Strogin 2, Strogin 4, Selligueanin A, Dihydroquercetin-3-acetat, Perillartin, Telosmosid A15, Periandrin I-V, Phyllodulcin, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Hernandulcin, Glycyrrhetinsäure, Balansin A, Balansin B;
(vii) Die natürlich vorkommenden Süßstoffe aus der Gruppe (i) können in der erfindungsgemäßen Aromamischungen auch eingesetzt werden in Form von Extrakten oder angereicherten Fraktionen dieser Extrakte, insbesondere Thaumatococcus-Extrakte (Katemfestaude), Extrakte aus *Stevia* ssp. (insbesondere *Stevia rebaudiana*), Swingle-Extrakt (*Momordica* bzw. *Siratia grosvenorii,* Luo-Han-Guo), Extrakte aus Süßholzwurzel, auch *Glycerrhyzia* ssp. (insb. *Glycerrhyzia glabra*), *Rubus* ssp. (insbesondere *Rubus suavissimus*), Citrus-Extrakte, Extrakte aus *Hydrangea macrophylla* var. *thunbergii* Makino, Extrakte aus *Lippia dulcis,* Extrakte aus *Mycetia balansae sowie* entsprechend angereicherte Fraktionen dieser Extrakte.
(viii) Süß schmeckende Aminosäuren (z.B. Glycin, D-Leucin, D-Threonin, D-Asparagin, D-Phenylalanin, D-tryptophan, L-Prolin);
(viii) Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (*Glycyrrhizza glabra* ssp.), *Lippia dulcis* Extrakte, *Momordica* ssp. Extrakte oder
(ix) Fruchtzubereitungen oder Extrakte z.B. aus *Momordica grosvenori* [Luo Han Guo], *Hydrangea dulcis* wie in EP 2,298,084-B1 beschrieben oder *Stevia* ssp. (z.B. *Stevia rebaudiana*) Extrakte
(x) Enzymatisch behandelte glycosidische Süßstoffe oder Extrakte aus *Stevia* ssp. oder *Rubus* ssp., wie in WO 2015 062,998 eschrieben.
(xi) Extrakte oder isolierte Balansine aus *Mycetia balansae* wie in WO 2012 164,062 beschrieben.

Dabei ist es aus anwendungstechnischen Gründen bevorzugt, dass diese süßen Stoffe oder deren Mischungen in der oralen Zubereitungen eine Süßintensität erreichen, die mindestens einer Süßintensität einer vergleichenden oralen Zubereitung entspricht, die nur 2 % Saccharose ohne weitere süße Stoffe enthält.

### AROMASTOFFE

In einer bevorzugten Ausführungsform der vorliegenden Erfindung enthalten diese als optionale Komponente (d) Aromastoffe.

Geeignete Aromastoffe können ausgewählt sein aus der folgenden Liste: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, delta-Decalacton, Diacetyl, Dihydrocumarin, Dimethylanthranilat, Dodecalacton, Essigsäure, Ethylacetat, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, , Geraniol, Geranylacetat, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-3-Hexenylacetat, cis-3-Hexenylcapronat, trans-2-Hexenylcapronat, cis-3-Hexenylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylisovalerianat, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureisoamylester, 3-Methyl-buttersäureethylester, Ethyl-3-methyl-3-phenylglycidat, Ethyl-2-trans-4-cis-decadienoat, 1,1-Dimethoxy-2,2,5-trimethyl-4-hexan, Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Typische Beispiele sind weiterhin: alpha-Ionon, beta-Ionon, Benzaldehyd, Citral, Damascon, delta-Decalacton, Diacetyl, Dimethylanthranilat, Dodecalacton, Essigsäure, Ethylacetat, Ethylbutyrat, Ethylfuraneol, Ethylisobutyrat, Ethylisovalerianat, Ethylmethylbutyrat, Ethylpropionat, Geraniol, Geranylacetat, Heliotropin, trans-2-Hexenal, cis-3-Hexenol, cis-3-Hexenylacetat, para-Hydroxybenzylaceton, Isobutyraldehyd, Linalool, Linalylacetat, 3,2,2-Methylcyclopentenolon, 2-Methylmethylbutyrat, Nerol, Nerylacetat, Vanillin, Ethylvanillin, 2,5-Dimethyl-4-hydroxy-3(2H)-furanon, Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureisoamylester, 3-Methyl-buttersäureethylester, Ethyl-2-trans-4-cis-decadienoat, (E,E)-2,4-Decadienal, Guajakol, Zimtaldehyd sowie (hier nicht explizit genannte) Stereoisomere, Enantiomere, Stellungsisomere, Diastereomere, cis/trans-Isomere bzw. Epimere dieser Substanzen.

Bei der Komponente (d) kann es sich auch modulierende Aroma- und/oder Geschmackstoffe handeln, die vorzugsweise ausgewählt sind aus der Gruppe bestehend aus Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Di-hydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)-amid, 2,4-Dihydroxy-benzoesäure-*N*-(4-hydroxy-3-methoxybenzyl)amid-Mono-natriumsalz, 2,4-Dihydroxy-benzoesäure-*N*-2-(4-hydroxy-3-methoxyphenyl)-ethyl-amid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxy-benzoesäurevanillylamid; Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]); Diacetyltrimeren; γ-Aminobuttersäuren und Divanillinen; Bicyclo[4.1.0]heptan-7-carbonsäureamide; Cyclopropancarbonsäure(3-methyl-cyclohexyl)amide, aromatische *Neo*-Menthylamide; Geranylaminederivate der Oxalsäure sowie Neomenthylderivate.

### STOFFGEMISCHE

Die erfindungsgemäßen Stoffgemische können die Komponenten (a) und (b) im Gewichtsverhältnis von etwa 99:1 bis etwa 1:99 enthalten. Besonders bevorzugt ist es dabei, wenn die Komponente (b) ein Gemisch von S- und R-Naringenin darstellt, beispielsweise im Gewichtsverhältnis 99:1 bis 80:20 und insbesondere etwa 97:3 bis etwa 90:10.

Weiterhin bevorzugt sind Stoffgemische, enthaltend
(a) etwa 1 bis etwa 25 Gew.-% Phloretin
(b) etwa 1 bis etwa 25 Gew.-% Naringenin
(c) etwa 1 bis etwa 50 Gew.-% mindestens einen weiteren süßen Stoff, der von Phloretin und Naringenin verschieden ist,
mit der Maßgabe, dass sich die Mengenangaben gegebenenfalls unter Zugabe von Aromastoffen, die die optionale Komponente (d) bilden zu 100 Gew.-% ergänzen.

Eine typische Zusammensetzung der Mischungen bezogen auf die Komponenten (a) und (b) sieht daher beispielsweise wie folgt aus:
(a) etwa 5 bis etwa 99,8, vorzugsweise etwa 25 bis 75, besonders bevorzugt 40 bis 60 Gew.-% Phloretin,
(b1) etwa 0,1 bis etwa 94,9, vorzugsweise etwa 25 bis 75, besonders bevorzugt 40 bis 60 Gew.-% S-Naringenin, wobei eines der Naringenin-Enantiomere in Bezug auf den Gesamtgehalt an Naringenin zu 100:1 bis 1:100 vorliegen können,
(b2) etwa 0,1 bis etwa 94,9, vorzugsweise etwa 25 bis 75, besonders bevorzugt 40 bis 60 Gew.-% R-Naringenin, wobei eines der der Naringenin-Enantiomere in Bezug auf Gesamtgehalt an Naringenin zu 100:1 bis 1:100 vorliegen können,
wobei die Maßgabe besteht, dass sich die Mengenangaben zu 100 Gew.-% ergänzen.

Besonders vorteilhaft sind indes Mischungen, die der vorhergehenden Zusammensetzung folgen und dabei
(a) etwa 5 bis etwa 90, vorzugsweise zwischen 25 bis 75, besonders bevorzugt 45 bis 55 Gew.-% Phloretin,
(b1) zwischen 10 bis etwa 85, vorzugsweise zwischen 30 bis 70, besonders bevorzugt 45 bis 55 Gew.-% S-Naringenin,
(b2) zwischen 0,1 bis etwa 85, vorzugsweise zwischen 0,1 bis 50, besonders bevorzugt 0,1 bis 1 Gew.-% R-Naringenin,
enthalten sind.

Ganz besonders vorteilhaft sind indes Mischungen, die der vorhergehenden Zusammensetzung folgen und dabei
(a) mehr als 30 Gew.-% Phloretin,
(b1) mehr als 30 Gew.-% S-Naringenin,
(b2) weniger als 1 Gew.-% R-Naringenin
enthalten.

Zu den bevorzugten Ausführungsformen gehören auch Stoffgemische, die die Komponenten (a+b) und (c) im Gewichtsverhältnis von etwa 20:80 bis 80:20, vorzugsweise etwa 40:60 bis 60:40 und insbesondere etwa 50:50 enthalten.

Diese Mischungen lassen sich nach dem weiter oben beschriebenen enzymatischen oder fermentativen Verfahren der EP 2692729 aus einem oder mehreren der folgenden Flavonoide erhalten: Naringenin, Naringin, Narirutin. Soweit diese Schriften die Herstellung der Mischungen beschreiben, wird ihr Inhalt hiermit durch Bezugnahme eingeschlossen.

### ORALE ZUBEREITUNGEN

Ein weiterer Gegenstand der Erfindung betrifft orale Zubereitungen, speziell Nahrungsmittel die die oben beschriebenen Stoffgemische mit oder ohne Gehalt an Aromastoffen enthalten, und zwar vorzugsweise in Mengen von 0,00001 bis etwa 2 Gew.-%. Weitere bevorzugte Zusatzmengen sind 0,0001 bis etwa 1,5 Gew.-%, insbesondere 0,001 bis etwa 1 Gew.-%, weiter bevorzugt etwa 0,01 bis etwa 0,5 Gew.-% und ganz besonders bevorzugt etwa 0,05 bis 0,1 Gew.-%

Insbesondere enthalten die oralen Zubereitungen im Allgemeinen und die Nahrungsmittel im Besonderen etwa 10 bis etwa 100 ppm Phloretin und etwa 10 bis etwa 100 ppm Naringenin.

Typische Beispiele für orale Zubereitungen umfassen:
- Backwaren, beispielsweise Brot, Trockenkekse, Kuchen, sonstiges Gebäck,
- Süßwaren (beispielsweise Schokoladen, Schokoladenriegelprodukte, sonstige Riegelprodukte, Fruchtgummi, Hart- und Weichkaramellen, Kaugummi),
- alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte,
- Frucht- oder Gemüsesaftzubereitungen,
- Instantgetränke (beispielsweise Instant-Kakao-Getränke, Instant-Tee-Getränke, Instant-Kaffeegetränke, Instant-Fruchtgetränke),
- Fleischprodukte (beispielsweise Schinken, Frischwurst- oder Rohwurstzubereitungen, gewürzte oder marinierte Frisch- oder Pökelfleischprodukte),
- Eier oder Eiprodukte (Trockenei, Eiweiß, Eigelb), Getreideprodukte (beispielsweise Frühstückscerealien, Müsliriegel, vorgegarte Fertigreis-Produkte),
- Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte),
- Produkte aus Sojaprotein oder anderen Sojabohnen-Fraktionen (beispielsweise Sojamilch und daraus gefertigte Produkte, Fruchtgetränke mit Sojaprotein, Sojalecithinhaltige Zubereitungen, fermentierte Produkte wie Tofu oder Tempe oder daraus gefertigte Produkte),
- Produkte aus anderen pflanzlichen Proteinquellen, beispielsweise Haferprotein-Getränke,
- Fruchtzubereitungen (beispielsweise Konfitüren, Fruchteis, Fruchtsoßen, Fruchtfüllungen),
- Gemüsezubereitungen (beispielsweise Ketchup, Soßen, Trockengemüse, Tiefkühlgemüse, vorgegarte Gemüse, eingekochte Gemüse),
- Knabberartikel (beispielsweise gebackene oder frittierte Kartoffelchips oder Kartoffelteigprodukte, Extrudate auf Mais- oder Erdnussbasis),
- Produkte auf Fett- und Ölbasis oder Emulsionen derselben (beispielsweise Mayonnaise, Remoulade, Dressings),
- sonstige Fertiggerichte und Suppen (beispielsweise Trockensuppen, Instant-Suppen, vorgegarte Suppen),
- Gewürze, Würzmischungen sowie insbesondere Aufstreuwürzungen (englisch: Seasonings), die beispielsweise im Snackbereich Anwendung finden.

Besonders bevorzugt sind hier Süßwaren, Milchprodukte und ganz besonders nicht-alkoholische Getränke, wobei gesüßte Getränke bevorzugt sind.

Des Weiteren kann es sich bei den oralen Zubereitungen auch Zahn- und Mundpflegprodukte, wie beispielsweise Zahnpasten, Mundwässer oder Kaugummis handeln.

### NAHRUNGSMITTELZUSATZSTOFFE

In einer weiteren Ausführungsform der vorliegenden Erfindung ist vorgesehen, dass die oralen Zubereitungen und speziell die Nahrungsmittel weitere Hilfs- und Zusatzstoffe, wie zum Beispiel Starterkulturen, Emulgatoren, Verdickungsmittel, Lebensmittelsäuren, Säureregulatoren, Vitamine, Antioxidantien, Lebensmittelfarbstoffe und dergleichen in Mengen von beispielsweise etwa 0,1 bis etwa 10 Gew.-%, vorzugsweise etwa 0,5 bis etwa 8 Gew.-%, insbesondere etwa 1 bis etwa 5 Gew.-% und besonders bevorzugt etwa 2 bis etwa 3 Gew.-% enthalten können.

### Emulgatoren

Emulgatoren zeichnen sich durch die wichtige Eigenschaft aus, sowohl in Wasser als auch in Fett löslich zu sein. Emulgatoren bestehen meist aus einem fettlöslichen und einem wasserlöslichen Teil. Sie kommen immer dann zum Einsatz, wenn Wasser und Öl zu einer beständigen, homogenen Vermischung gebracht werden sollen.

Geeignete Emulgatoren, die in der lebensmittelverarbeitenden Industrie verwendet werden sind ausgewählt aus: Ascorbylpalmitat (E 304) Lezithin (E 322) Phosphorsäure (E 338) Natriumphosphat (E 339) Kaliumphosphat (E 340) Kalziumphosphat (E 341) Magnesiumorthophosphat (E 343) Propylenglykolalginat (E 405) Polyoxyethylen(8)stearat (E 430) Polyoxyethylenstearat (E 431) Ammoniumphosphatide (E 442) Natriumphosphat und Kaliumphosphat (E 450) Natriumsalze der Speisefettsäuren (E 470 a) Mono- und Diglyceride von Speisefettsäuren (E 471) Essigsäuremonoglyceride (E 472 a) Milchsäuremonoglyceride (E 472 b) Zitronensäuremonoglyceride (E 472 c) Weinsäuremonoglyceride (E 472 d) Diacetylweinsäuremonoglyceride (E 472 e) Zuckerester von Speisefettsäuren (E 473) Zuckerglyceride (E 474) Polyglyceride von Speisefettsäuren (E 475) Polyglycerin-Polyricinoleat (E 476) Propylenglykolester von Speisefettsäuren (E 477) Natriumstearoyllaktylat (E 481) Calciumstearoyl-2-lactylat (E 482) Stearyltartrat (E 483) Sorbitanmonostearat (E 491) Stearinsäure (E 570).

### Verdickungsmittel

Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder Ionenbeziehungen miteinander interagieren können. Extremfälle von Dickungsmitteln sind Schichtsilikate (Bentonite, Hectorite) oder hydratisierte SiO₂-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:
- E 400 -: Alginsäure
- E 401 -: Natriumalginat
- E 402 -: Kaliumalginat
- E 403 -: Ammoniumalginat
- E 404 -: Calciumalginat
- E 405 -: Propylenglycolalginat
- E 406 -: Agar Agar
- E 407 -: Carrgeen, Furcelleran
- E 407 -: Johannisbrotkernmehl
- E 412 -: Guarkernmehl
- E 413 -: Traganth
- E 414 -: Gummi arabicum
- E 415 -: Xanthan
- E 416 -: Karaya (IndischerTraganth)
- E 417 -: Tarakernmehl (Peruanisches Johannisbrotkernmehl)
- E 418 -: Gellan
- E 440 -: Pektin, Opekta
- E 440ii -: Amidiertes Pektin
- E 460 -: Mikrokristalline Cellulose, Cellulosepulver
- E 461 -: Methylcellulose
- E 462 -: Ethylcellulose
- E 463 -: Hydroxypropylcellulose
- E 465 -: Methylethylcellulose
- E 466 -: Carboxymethylcellulose, Natriumcarboxymethylcellulose

### Lebensmittelsäuren

Die Nahrungsmittel können Carbonsäuren enthalten. Säuren im Sinne der Erfindung sind bevorzugt in Lebensmitteln zulässige Säuren, insbesondere die hier genannten:
- E 260 -: Essigsäure
- E 270 -: Milchsäure
- E 290 -: Kohlendioxid
- E 296 -: Apfelsäure
- E 297 -: Fumarsäure
- E 330 -: Citronensäure
- E 331 -: Natriumcitrat
- E 332 -: Kaliumcitrat
- E 333 -: Calciumcitrat
- E 334 -: Weinsäure
- E 335 -: Natriumtartrat
- E 336 -: Kaliumtartrat
- E 337 -: Natrium-Kaliumtartrat
- E 338 -: Phosphorsäure
- E 353 -: Metaweinsäure
- E 354 -: Calciumtartrat
- E 355 -: Adipinsäure
- E 363 -: Bernsteinsäure
- E 380 -: Triammoniumcitrat
- E 513 -: Schwefelsäure
- E 574 -: Gluconsäure
- E 575 -: Glucono-delta-Lacton

### Säureregulatoren

Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:
- E 170: - Calciumcarbonat
- E 260-263: - Essigsäure und Acetate
- E 270: - Milchsäure
- E 296: - Äpfelsäure
- E 297: - Fumarsäure
- E 325-327: - Lactate (Milchsäure)
- E 330-333: - Citronensäure und Citrate
- E 334-337: - Weinsäure und Tartrate
- E 339-341: - Orthophosphate
- E 350-352: - Malate (Äpfelsäure)
- E 450-452: - Di-, Tri- und Polyphosphate
- E 500-504: - Carbonate (Kohlensäure)
- E 507: - Salzsäure und ChlorideE 513-517Schwefelsäure und Sulfate
- E 524-528: - Hydroxide
- E 529-530: - Oxide
- E 355-357: - Adipinsäure und Adipate
- E 574-578: - Gluconsäure und Gluconate

### Vitamine

In einer weiteren Ausführungsform der vorliegenden Erfindung können die Nahrungsmittelzusatzstoffe als weitere fakultative Gruppe von Zusatzstoffen Vitamine enthalten. Vitamine verfügen über unterschiedlichste biochemische Wirkungsweisen. Einige wirken ähnlich wie Hormone und regulieren den Mineralmetabolismus (z.B. Vitamin D), oder wirken auf das Wachstum von Zellen und Gewebe sowie die Zelldifferenzierung (z.B. einige Formen des Vitamin A). Andere stellen Antioxidantien dar (z.B. Vitamin E und unter bestimmten Umständen auch Vitamin C). Die größte Zahl von Vitaminen (z.B. die B-Vitamine) stellen Vorstufen für enzymatische Co-Faktoren dar, die Enzyme dabei unterstützen, bestimmte Prozesse im Metabolismus zu katalysieren. In diesem Zusammenhang können Vitamin mitunter eng an die Enzyme gebunden sein, beispielsweise als Teil der prostetischen Gruppe: ein Beispiel hierfür ist Biotin, das ein Teil des Enzyms ist, welches für den Aufbau von Fettsäuren verantwortlich ist. Vitamine können andererseits auch weniger stark gebunden sein und dann als Co-Katalysatoren wirken, beispielsweise als Gruppen, die sich leicht abspalten lassen und chemische Gruppen oder Elektronen zwischen den Molekülen transportieren. So transportiert beispielsweise Folsäure Methyl-, Formyl- und Methylengruppen in die Zelle. Obwohl ihre Unterstützung in Enzym-Substrat-Reaktionen wohl bekannt ist, sind auch ihre übrigen Eigenschaften für den Körper von großer Bedeutung.

Im Rahmen der vorliegenden Erfindung kommen als Vitamine Stoffe in Betracht, die ausgewählt sind aus der Gruppe bestehend aus
- Vitamin A (Retinol, Retinal, Betakarotin),
- Vitamin B₁ (Thiamin),
- Vitamin B₂ (Rioflavin),
- Vitamin B₃ (Niacin, Niacinamid),
- Vitamin B₅ (Panthothensäure),
- Vitamin B₆ (Pyridoxin, Pyridoxamin, Paridoxal),
- Vitamin B₇ (Biotin),
- Vitamin B₉ (Folsäure, Folinsäure),
- Vitamin B₁₂ (Cyanobalamin, Hydroxycobalmin, Methylcobalmin),
- Vitamin C (Ascorbinsäure),
- Vitamin D (Cholecalciferol),
- Vitamin E (Tocopherole, Tocotrienole) und
- Vitamin K (Phyllochinon, Menachinon).

Die bevorzugten Vitamine sind neben der Ascorbinsäure die Gruppe der Tocopherole.

### Antioxidantien

In der Lebensmittelindustrie werden sowohl natürliche als auch künstliche Antioxidationsmittel verwendet. Natürliche und künstliche Antioxidantien unterscheiden sich in erster Linie dadurch, dass erstere natürlich in der Nahrung vorkommen und letztere künstlich hergestellt werden. So werden natürliche Antioxidationsmittel, so sie als Lebensmittelzusatzstoff eingesetzt werden sollen, beispielsweise aus Pflanzenölen gewonnen. Vitamin E - auch als Tocopherol bekannt - wird beispielsweise häufig aus Sojaöl hergestellt. Synthetische Antioxidantien wie das Propylgallat, das Octylgallat und das Dodecylgallat werden dagegen durch chemische Synthese gewonnen. Die Gallate können bei empfindlichen Personen Allergien auslösen. Weitere einsetzbare Antioxidantien in Zusammensetzungen der vorliegenden Erfindung sind: Schwefeldioxid, E 220 Sulfite Natriumsulfit, E 221 Natriumhydrogensulfit, E 222 Natriumdisulfit, E 223 Kaliumdisulfit, E 224 Kalziumsulfit, E 226 Kalziumhydrogensulfit, E 227 Kaliumhydrogensulfit, E 228 Milchsäure, E 270 Ascorbinsäure, E 300 Natrium-L-Ascorbat, E 301 Calcium-L-Ascorbat, E 302 Ascorbinsäureester, E 304 Tocopherol, E 306 Alpha-Tocopherol, E 307 Gamma-Tocopherol, E 308 Delta-Tocopherol, E 309 Propylgallat, E 310 Octygallat, E 311 Dodecylgallat, E 312 Isoascorbinsäure, E 315 Natriumisoascorbat, E 316 tertiär-Butylhydrochinon (TBHQ), E 319 Butylhydroxianisol, E 320 Butylhydroxitoluol, E 321 Lecithin, E 322 Citronensäure, E 330 Salze der Zitronensäure (E 331 & E 332) Natriumzitrat, E 331 Kaliumzitrat, E 332 Calcium-Dinatrium-EDTA, E 385 Diphosphate, E 450 Dinatriumdiphosphat, E 450a Trinatriumdiphosphat, E 450b Tetranatriumdiphosphat, E 450c Dikaliumdiphosphat, E 450d Trekaliumdiphosphat, E 450e Dikalziumdiphosphat, E 450f Kalziumdihydrogendiphosphst, E 450g Triphosphate, E 451 Pentanatriumtriphosphat, E451a Pentakaliumtriphosphat, E 451b Polyphosphat, E 452 Natriumpolyphosphat, E 452a Kaliumpolyphosphat, E 452b Natriumkalziumpolyphosphat, E 452c Kalziumpolyphosphat, E 452d Zinn-II-Chlorid, E 512.

### Lebensmittelfarbstoffe

Lebensmittelfarbstoffe oder kurz Farbstoffe sind Lebensmittelzusatzstoffe zum Färben von Lebensmittel. Farbstoffe werden in die Gruppen der natürlichen Farbstoffe und synthetischen Farbstoffe unterteilt. Die naturidentischen Farbstoffe sind ebenfalls synthetischen Ursprungs. Die natur-identische Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotinsäure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### MUND- UND ZAHNPFLEGEMITTEL

Erfindungsgemäße oral konsumierbare süß schmeckende Produkte können auch der Mund- und Zahnreinigung und-pflege dienen. Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stiviaextrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;

wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### KAUGUMMIS

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als süße Stoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharide, die als süße Stoffe wirken sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Rebaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### GEWERBLICHE ANWENDBARKEIT

Weiterhin beschrieben ist ein erstes Verfahren zur Verbesserung der Geschmacksqualität Phloretin in einem Nahrungsmittel, welches sich dadurch auszeichnet, dass es die folgenden Schritte umfasst:
(a) Bereitstellen einer Nahrungsmittelbasis, die einen süßen Stoff und Phloretin enthält und
(b) Versetzen der Basis mit einer Menge an Naringenin, die eine Verbesserung der Geschmacksqualität des Phloretins bewirkt.

Auch beschrieben wird ein zweites Verfahren zur Verbesserung der Geschmacksqualität von Phloretin in einem Nahrungsmittel, welches sich dadurch auszeichnet, dass es die folgenden Schritte umfasst:
(a) Bereitstellen einer Nahrungsmittelbasis, die einen süßen Stoff enthält und
(b) Versetzen der Basis mit einer wirksamen Menge an Phloretin und Naringenin, wobei die Menge des Naringenins so bemessen ist, dass sie eine Verbesserung der Geschmacksqualität des Phloretins bewirkt.

Unter einer wirksamen Menge an Naringenin ist jede Menge zu verstehen, die geeignet ist, den bitteren und adstringenten Geschmackseindruck des Phloretins zu überdecken oder zu kompensieren. Entsprechende Mengen kann der Fachmann im Zweifelsfall durch einfaches Testen ohne weitere Hinweise ermitteln, ohne dazu erfinderisch tätig werden zu müssen. In der Regel entspricht eine wirksame Menge Naringenin etwa einem Zehntel bis etwa des Zehnfachen der Menge an Phloretin.

Der zweite Gegenstand der Erfindung betrifft schließlich die Verwendung von Naringenin zur Verbesserung der Geschmacksqualität von Phloretin, speziell wenn Phloretin in einem Nahrungsmittel zugegen ist.

### BEISPIELE

### BEISPIELE 1-2, VERGLEICHSBEISPIELE V1-V2

Mit einem Panel bestehend aus 9 erfahrenen Testern wurde eine Blindverkostung von verschiedenen Testformulierungen durchgeführt. Dabei sollten die folgenden Eigenschaften
- Sofortsüße ("Onset sweetness")
- Süßintensität ("Sweetness itensity")
- Fülle des Zuckergeschmackes ("Full body, sugar like taste")
- Bitterkeit ("bitter")
- Adstringenz ("adstringent")
auf einer Skala von (1) = gering bis (6) stark ausgeprägt beurteilt werden. Die Ergebnisse sind in der nachfolgenden **Tabelle 1** zusammengefasst.

**Tabelle 1**

| Verkostungsergebnisse | | | | | | |
|---|---|---|---|---|---|---|
| **Bsp.** | **Zusammensetzung** | **Onset sweetness** | **Sweetness intensity** | **Full body sugar like taste** | **Bitter** | **Adstringent** |
| C | Basis (5 % Zucker) | 4,1 | 4,6 | 4,6 | 0,7 | 1,0 |
| V1 | Basis | 4,4 | 4,8 | 4,5 | 1,0 | 1,0 |
| | + 15 ppm Phloretin | | | | | |
| V2 | Basis | 4,8 | 5,8 | 4,1 | 2,3 | 2,5 |
| | + 30 ppm Phloretin | | | | | |
| 1 | Basis | 5,2 | 6,0 | 4,6 | 1,6 | 1,7 |
| | + 15 ppm Phloretin | | | | | |
| | + 15 ppm R/S-Naringenin | | | | | |
| 2 | Basis | 5,3 | 6,0 | 4,8 | 1,8 | 2,3 |
| | + 30 ppm Phloretin | | | | | |
| | + 30 ppm R/S-Naringenin | | | | | |

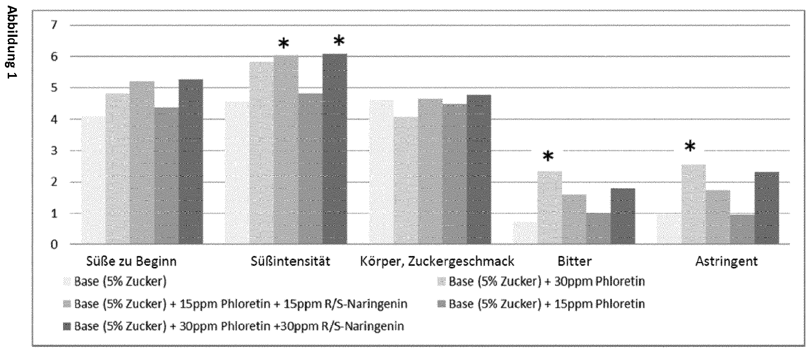

Eine graphische Darstellung der Verkostungsergebnisse ist in der nachfolgenden Abbildung 1 wiedergegeben. Die mit (*) gekennzeichneten Balken zeigen signifikante Unterschiede zur Kontrollbasis (C) (t-test, α=1)

Die nachfolgenden **Tabellen A bis G** geben Beispielformulierungen für unterschiedliche Endprodukte wieder.

**Tabelle A**

| Aromazubereitungen | | | | | | | | | | |
|---|---|---|---|---|---|---|---|---|---|---|
| **Zusammensetzung** | **A** | **B** | **C** | **D** | **E** | **F** | **G** | **H** | **I** | **J** |
| R-Naringenin | 10 | 20 | 10 | | | | | 10 | | |
| S-Naringenin | | | | 20 | 15 | | 7,5 | | | |
| R-/S-Naringenin | | | | | | 10 | | | 20 | 20 |
| Hesperetin aus Citrus (racemisch, >80 %) | 2 | | | | | | | | 2 | |
| (S)-Hesperetin aus Honigbusch (> 95%) | | | 1 | | | | 1,5 | | | |
| Phloretin | 10 | 10 | 2 | 5 | | 8 | 2 | 7 | 5 | 10 |
| Hesperetin | | 3 | | 3 | 1 | | | 2 | | 3 |
| 1-(2,4-Dihydroxy-phenyl)-3-(3-hydroxy-4-methoxyphenyl)-propan-1-on | | | | | | 2 | | | | |
| 7,3-Dihydroxy-4'methoxyflavan | | | | | 2 | | | | | |
| 5-Hydroxy-4-(4-hydroxy-3-methoxy-phenyl)-7-methoxy-2-chromanon | | | | | | 2 | | | | |
| Glycerin | 10 | 20 | | 30 | | 20 | 15 | 30 | 10 | 20 |
| Gummi arabicum-Lösung (20%) | | | | | | | | 10 | | |
| 1,2-Propylenglycol | Ad 100 | | | | | | | | | |

**Tabelle B**

| Erfrischungsgetränke (Mengenangaben als Gew.-%) | | | | | | | |
|---|---|---|---|---|---|---|---|
| **Inhaltsstoffe** | **A** | **B** | **C** | **D** | **E** | **F** | **G** |
| Sucrose | 10 | 10 | 7 | - | - | 8 | 7 |
| Glucose/Fructose Sirup | - | - | - | - | 10 | - | - |
| Phloretin/Naringenin Mischung A aus Beispiel 1 | 0,003 | 0,005 | 0,003 | 0,01 | 0,0045 | 0,0025 | 0,001 |
| Zitronensäure | 0,15 | 0,15 | 0,06 | 0,15 | 0,15 | 0,15 | 0,15 |
| Phosphorsäure | - | - | 0,07 | - | - | - | - |
| Zuckercoleur | - | - | 0,14 | - | - | - | - |
| Koffein | - | - | 0,01 | - | - | - | - |
| Zitrusaroma | 0,1 | 0,05 | - | 0,1 | 0,1 | 0,1 | 0,1 |
| Limonenaroma | - | 0,05 | - | - | - | - | - |
| Getränkeemulsion Typ "Cola" | - | - | 0,05 | - | - | - | - |
| Hesperetin | 0,00075 | 0,0012 | 0,00075 | 0,0012 | 0,00075 | 0,00075 | 0,01 |
| Homoeriodictyol-Na | - | - | 0,005 | 0,005 | - | - | - |
| Wasser | | | | Ad 100 | | | |

Herstellung: Die Zutaten werden in der angegebenen Reihenfolge gemischt und mit Wasser auf 100 % aufgefüllt. Anschließend werden die Mischungen in Glasflaschen gefüllt und karbonisiert.

**Tabelle C**

| Hartkaramellen (Mengenangaben als Gew.-%) | | | | |
|---|---|---|---|---|
| **Inhaltsstoffe** | **A** | **B** | **C** | **D** |
| Zucker | 74,50 | - | - | - |
| Palatinit, Type M | - | 74,00 | 75,50 | 75,00 |
| Zitronensäure | 0,5 | 1,0 | 0,5 | - |
| Farbstoff, gelb | - | 0,01 | - | - |
| Farbstoff, rot | - | - | 0,01 | - |
| Farbstoff, blau | 0,01 | - | - | 0,01 |
| Pfefferminzaroma | 0,1 | - | - | 0,1 |
| Zitrusaroma | - | 0,1 | - | - |
| Rote Beeren-Aroma | - | - | 0,1 | - |
| Phloretin/Naringenin Mischung A aus Beispiel 1 | 0,005 | 0,0045 | 0,006 | 0,003 |
| Balansin A | - | 0,005 | 0,010 | 0,005 |
| Hesperetin | 0,00075 | 0,001 | 0,0005 | 0,0004 |
| Wasser | ad 100 | ad 100 | ad 100 | ad 100 |

**Tabelle D**

| Joghurt mit niedrigem Fettgehalt (Mengenangaben als Gew.-%) | | | | |
|---|---|---|---|---|
| **Inhaltsstoffe** | **A** | **B** | **C** | **D** |
| Sucrose | 10 | 8 | 6 | - |
| Sucralose | - | 0,02 | - | 0,2 |
| Rebaudiosid A > 95 % | - | - | 0,025 | - |
| Saccharin | - | - | - | 0,3 |
| Phloretin/Naringenin Mischung A aus Beispiel 1 | 0,005 | 0,0045 | 0,01 | 0,050 |
| Sauerkirsch-Extrakt nach Beispiel 1 | 0,2 | 0,1 | 0,2 | 0,2 |
| Hesperetin | 0,00075 | 0,001 | 0,003 | 0,002 |
| Homoeriodictyol-Natriumsalz | - | - | - | 0,005 |
| Joghurt, 0,1 % Fett | zu 100 % auffüllen | | | |

**Tabelle E**

| Fruchtgummis (Mengenangaben als Gew.-%) | | |
|---|---|---|
| **Inhaltsstoffe** | **A** | **B** |
| Saccharose | 34,50 | 8,20 |
| Glucosesirup, DE 40 | 31,89 | 30,09 |
| Phloretin/Naringenin Mischung A aus Beispiel 1 | 0,005 | 0,0045 |
| Iso Sirup C* Tru Sweet 01750 (Cerestar GmbH) | 1,50 | 2,10 |
| Gelatine 240 Bloom | 8,20 | 9,40 |
| Polydextrose (Litesse® Ultra, Danisco Cultor GmbH) | - | 24,40 |
| Farbstoff | 0,01 | 0,01 |
| Zitrusaroma | 0,20 | - |
| Kirscharoma | - | 0,10 |
| Hesperetin | 0,0075 | 0,0015 |
| Wasser | ad 100 | ad 100 |

**Tabelle F**

| Zuckerfreies Kaugummi (Mengenangaben als Gew.-%) | |
|---|---|
| **Inhaltsstoffe** | **Gehalt** |
| Kaugummibasis | 30.00 |
| Sorbitolpulver | Ad 100 |
| Phloretin/Naringenin Mischung A aus Beispiel 1 | 0,05 |
| Palatinit | 9.50 |
| Xylitol | 2.00 |
| Mannitol | 3.00 |
| Aspartam | 0.10 |
| Acesulfam K | 0.10 |
| Emulgum / Emulgator | 0.30 |
| Sorbitol 70 %. in Wasser | 14.00 |
| Glycerin | 1.00 |
| Pfefferminzaroma | 1.50 |
| Hesperetin | 0,01 |

**Tabelle G**

| Pudding (Mengenangaben in g) | | | |
|---|---|---|---|
| **Inhaltsstoffe** | **A** | **B** | **C** |
| Maisstärke | 38 | 38 | 38 |
| Zucker | 38 | 30 | 22,8 |
| Phloretin/Naringenin Mischung A aus Beispiel 1 | - | 0,05 | 0,08 |
| Hesperetin | - | 0,01 | 0,03 |
| Vanille-Aroma (Symrise) | 0,2 | 0,2 | 0,2 |
| Chinolin gelb | 0,02 | 0,02 | 0,02 |
| Milch | 500ml | 500ml | 500ml |

## Patentansprüche

1. Stoffgemische, enthaltend
(a) Phloretin,
(b) Naringenin,
(c) mindestens einen weiteren süßen Stoff, der von Phloretin und Naringenin verschieden ist, sowie gegebenenfalls
(d) mindestens einen Aromastoff,
wobei die Komponenten (a) und (b) im Gewichtsverhältnis von 99:1 bis 1:99 enthalten sind.

2. Stoffgemische nach Anspruch 1, **dadurch gekennzeichnet, dass** die süßen Stoffe, die die Komponente (c) bilden, ausgewählt sind aus der Gruppe, die gebildet wird von Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, Maltodextrine verschiedener Polymerisationsgrade sowie pflanzlicher Zubereitungen, die diese Stoffe enthalten.

3. Stoffgemische nach den Ansprüchen 1 und/oder 2, **dadurch gekennzeichnet, dass** die süßen Stoffe, die die Komponente (c) bilden, ausgewählt sind aus mindestens einer der folgenden Gruppen:
(i) enzymatisch hergestellte Stärke oder Zuckerhydrolysate wie Invertzucker oder Fructosesirup;
(ii) Fruchtkonzentrate;
(iii) Zuckeralkohole wie Erythritol, Threitol, Arabitol, Ribotol, Xylitol, Sorbitol, Mannitol, Dulcitol oder Lactitol;
(iv) süß schmeckende Proteine und Peptide wie Miraculin, Monellin, Thaumatin, Curculin oder Brazzein;
(v) synthetische Süßstoffe wie Magap, Natriumcyclamat, Acesulfam K, Neohesperidindihydrochalkon, Saccharin Natriumsalz, Aspartam, Superaspartam, Neotam, Alitam, Sucralose, Stevioside, Rebaudioside, Rubusoside, Suavioside, Mogroside, Lugduname, Carrelame, Sucrononate, Sucrooctate, Monatin, Phenylodulcin, Phyllodulcin, oder Abrusoside;
(vi) natürlich vorkommende Süßstoffe wie Steviosid, Steviolbiosid, Rebaudiosid A, weiteren Steviolglycosiden wie Rebaudiosid B, Rebaudiosid C, Rebaudiosid D, Rebaudiosid E, Rebaudiosid F, Rebaudiosid G, Rebaudiosid H, Dulcosid und/oder Rubusosid, Oslandin, Polypodosid A, Strogin 1, Strogin 2, Strogin 4, Selligueanin A, Dihydroquercetin-3-acetat, Perillartin, Telosmosid A15, Periandrin I-V, Phyllodulcin, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Hernandulcin, Glycyrrhetinsäure, Balansin A, Balansin B;
(vii) Extrakte oder angereicherte Fraktionen dieser Extrakte wie etwa Thaumatococcus-Extrakte (Katemfestaude), Extrakte aus *Stevia* ssp. (insbesondere *Stevia rebaudiana*), Swingle-Extrakt (*Momordica* bzw. *Siratia grosvenorii,* Luo-Han-Guo), Extrakte aus Süßholzwurzel, auch *Glycerrhyzia* ssp. (insb. *Glycerrhyzia glabra*), *Rubus* ssp. (insbesondere *Rubus suavissimus*), Citrus-Extrakte, Extrakte aus *Hydrangea macrophylla* var. *thunbergii* Makino, Extrakte aus *Lippia dulcis,* Extrakte aus *Mycetia balansae;*
(viii) süß schmeckende Aminosäuren (z.B. Glycin, D-Leucin, D-Threonin, D-Asparagin, D-Phenylalanin, D-tryptophan, L-Prolin);
(ix) süß schmeckende niedermolekulare Substanzen, wie Hernandulcin, Dihydrochalconglykoside, Glycyrrhizin, Glycerrhetinsäure, ihre Derivate und Salze, Extrakte von Lakritz (*Glycyrrhizza glabra* ssp.), *Lippia dulcis* Extrakte, *Momordica* ssp. Extrakte oder Fruchtzubereitungen oder Extrakte z.B. aus *Momordica grosvenori* [Luo Han Guo], *Hydrangea dulcis, Stevia* ssp. (z.B. *Stevia rebaudiana*);
(x) enzymatisch behandelte glycosidische Süßstoffe oder Extrakte aus *Stevia* ssp. oder *Rubus* ssp; sowie
(xi) Extrakte oder isolierte Balansine aus *Mycetia balansae.*

4. Stoffgemische nach mindestens einem der Ansprüche 1 bis 3, **dadurch gekennzeichnet, dass** sie als optionale Komponente (d) Aromastoffe enthalten, die ausgewählt sind aus der Gruppe, die gebildet wird von Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, delta-Decalacton, Diacetyl, Dihydrocumarin, Dimethylanthranilat, Dodecalacton, Essigsäure, Ethylacetat, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, , Geraniol, Geranylacetat, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-3-Hexenylacetat, cis-3-Hexenylcapronat, trans-2-Hexenylcapronat, cis-3-Hexenylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylisovalerianat, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-fu ranon, Essigsäureisoamylester, Buttersäureisoamylester, 3-Methyl-buttersäureethylester, Ethyl-3-methyl-3-phenylglycidat, Ethyl-2-trans-4-cis-decadienoat, 1,1-Dimethoxy-2,2,5-trimethyl-4-hexan, Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

5. Stoffgemische nach mindestens einem der Ansprüche 1 bis 4, **dadurch gekennzeichnet, dass** sie als optionale Komponente (d) Aromastoffe enthalten, die ausgewählt sind aus der Gruppe, die gebildet wird von alpha-Ionon, beta-Ionon, Benzaldehyd, Citral, Damascon, delta-Decalacton, Diacetyl, Dimethylanthranilat, Dodecalacton, Essigsäure, Ethylacetat, Ethylbutyrat, Ethylfuraneol, Ethylisobutyrat, Ethylisovalerianat, Ethylmethylbutyrat, Ethylpropionat, Geraniol, Geranylacetat, Heliotropin, trans-2-Hexenal, cis-3-Hexenol, cis-3-Hexenylacetat, para-Hydroxybenzylaceton, Isobutyraldehyd, Linalool, Linalylacetat, 3,2,2-Methylcyclopentenolon, 2-Methylmethylbutyrat, Nerol, Nerylacetat, Vanillin, Ethylvanillin, 2,5-Dimethyl-4-hydroxy-3(2H)-furanon, Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureisoamylester, 3-Methyl-buttersäureethylester, Ethyl-2-trans-4-cis-decadienoat, (E,E)-2,4-Decadienal, Guajakol, Zimtaldehyd sowie (hier nicht explizit genannte) Stereoisomere, Enantiomere, Stellungsisomere, Diastereomere, cis/trans-Isomere bzw. Epimere dieser Substanzen.

6. Stoffgemische nach mindestens einem der Ansprüche 1 bis 4, **dadurch gekennzeichnet, dass** sie als optionale Komponente (d) Aromastoffe enthalten, die ausgewählt sind aus der Gruppe, die gebildet wird von Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Di-hydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)-amid, 2,4-Dihydroxy-benzoesäure-*N*-(4-hydroxy-3-methoxybenzyl)amid-Mono-natriumsalz, 2,4-Dihydroxybenzoesäure-*N*-2-(4-hydroxy-3-methoxyphenyl)-ethyl-amid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxy-benzoesäurevanillylamid; Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]); Diacetyltrimeren; γ-Aminobuttersäuren und Divanillinen; Bicyclo[4.1.0]heptan-7-carbonsäureamide; Cyclopropancarbonsäure(3-methyl-cyclohexyl)amide, aromatische *Neo*-Menthylamide; Geranylaminederivate der Oxalsäure sowie Neomenthylderivate und deren Gemische.

7. Stoffgemische nach mindestens einem der Ansprüche 1 bis 6, **dadurch gekennzeichnet, dass** sie
(a) 1 bis 25 Gew.-% Phloretin,
(b) 1 bis 25 Gew.-% Naringenin, und
(c) 1 bis 50 Gew.-% mindestens einen weiteren süßen Stoff, der von Phloretin und Naringenin verschieden ist,
mit der Maßgabe enthalten, dass sich die Mengenangaben gegebenenfalls unter Zugabe von Aromastoffen, die die optionale Komponente (d) bilden, zu 100 Gew.-% ergänzen.

8. Stoffgemische nach mindestens einem der Ansprüche 1 bis 7, **dadurch gekennzeichnet, dass** sie bezogen auf die Komponenten (a) und (b) alleine
(a) 5 bis 99,8 Gew.-% Phloretin,
(b1) 0,1 bis 94,9 Gew.-% S-Naringenin, und
(b2) 0,1 bis 94,9 Gew.-% R-Naringenin, wobei
mit den Maßgaben enthalten, dass eines der Naringenin-Enantiomere in Bezug auf Gesamtgehalt an Naringenin im Gewichtsverhältnis zu 100:1 bis 1:100 vorliegen kann und sich die Mengenangaben zu 100 Gew.-% ergänzen.

9. Stoffgemische nach mindestens einem der Ansprüche 1 bis 8, **dadurch gekennzeichnet, dass** sie bezogen auf die Komponenten (a) und (b) alleine
(a) 5 bis 90 Gew.-% Phloretin,
(b1) 10 bis 85 Gew.-% S-Naringenin, und
(b2) 0,1 bis 85 Gew.-% R-Naringenin, wobei
mit der Maßgabe enthalten, dass sich die Mengenangaben zu 100 Gew.-% ergänzen.

10. Stoffgemische nach mindestens einem der Ansprüche 1 bis 9, **dadurch gekennzeichnet, dass** sie die Komponenten (a+b) und (c) im Gewichtsverhältnis von 20:80 bis 80:20 enthalten.

11. Orale Zubereitungen, enthaltend die Stoffgemische nach mindestens einem der Ansprüche 1 bis 10.

12. Zubereitungen nach Anspruch 11, **dadurch gekennzeichnet, dass** sie die Stoffgemische in Mengen von 0,00001 bis 2 Gew.-%. enthalten.

13. Verwendung von Naringenin zur Verbesserung der Geschmacksqualität von Phloretin.

## Claims

1. Substance mixtures, comprising
(a) phloretin,
(b) naringenin,
(c) at least one additional sweet substance, which is different from phloretin and naringenin, as well as optionally
(d) at least one flavouring,
wherein the components (a) and (b) are present in a weight ratio of 99:1 to 1:99.

2. The substance mixtures according to claim 1, **characterized in that** the sweet substances, which form the component (c), are selected from the group consisting of saccharose, trehalose, lactose, maltose, melizitose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyd, maltodextrine of different polymerisation grades as well as plant-based preparations, which contain these substances.

3. The substance mixtures according to claim 1 and/or 2, **characterized in that** the sweet substances, which form the component (c), are selected from at least one of the following groups:
(i) enzymatic produced starch or sugar hydrolysates such as invert sugar or fructose syrup;
(ii) fruit concentrates;
(iii) sugar alcohols such as erythritol, threitol, arabitol, ribotol, xylitol, sorbitol, mannitol, dulcitol or lactitol;
(iv) sweet tasting proteins and petides such as miraculin, monellin, thaumatin, curculin or brazzein;
(v) synthetic sweeteners such as magap, sodium cyclamat, acesulfam K, neohesperidin hydrochalcone, saccharin sodium salt, aspartame, super-aspartame , neotame, alitame, sucralose, steviosides, rebaudiosides, rubusosides, suaviosides, mogrosides, lugdunames, carrelames, sucronates, sucrooactates, monatines, phenylodulcines, phyllodulcines or abrusosides;
(vi) naturally occurring sweeteners such as stevioside, steviolbioside, rebaudioside A, further steviolglycosides such as rebaudioside B, rebaudioside C, rebaudioside D, rebaudioside E, rebaudioside F, rebadioses G, rebaudioside H, culcoside and/or rubusoside, oslandin, polypodoside A, strogin 1, strogin 2, strogin 4, selligueanin A, dihydroquercetine-3-acetate, perillartin, telosmoside A15, periandrin I-V, phyllodulcin, pterocaryosides, cyclocaryosides, mukurisiosides, trans-anethole, trans-cinnamaldehyde, bryosides, bryonosides, bryonodulcosides, carnosiflosides, scandenosides, gypenosides, trilobatin, phloridzine, dihydroflavanoles, hematoxylin, cyanin, chlorogenic acid, albiziasaponin, telosmosides, gaudichaudioside, mogrosides, hernandulcine, glycyrrhetinic acid, balansin A, balansin B;
(vii) extracts or enriched fractions of these extracts such as thaumatococcus extracts (Katemfestaude), extracts from *Stevia* ss. (especially *Stevia rebaudi*ana), swingle extract (*Momordica* or *Siratia grosvenorii,* Luo-Han-Guo), extracts from liquorice root, also *Glycerrhyzia* ssp. (especially *Glycerrhyzia glabra*), *Rubus* ssp. (especially *Rubus suavissimus*), citrus extracts, extracts from *Hydrangea macrophyll* var. *thunbergii* Makino, extacts from *Lippia dulcis,* extracts from *Mycetia balansae*;
(viii) sweet tasting amino acids (e.g. glycine, D-leucine, D-threonine, D-asparagine, D-phnylalanine, D-Tryptophane, L-proline);
(ix) sweet tasting low molecular weight substances, such as hernandulcin, dihydro chalconglykosides, glycyrrhizine, glycerrhetinic acid, derivates and salts thereof, extracts from liquorice (*Glycyrrhizza glabra* ssp.), *Lippia dulcis* extracts, *Momordica* ssp. extracts or fruit preparations or extracts e.g. from *Momordica grosvenori* [Luo Han Guo], *Hydrangea dulcis, Stevia* ssp. (e.g. *Stevia rebaudiana*);
(x) enzymatically treated glycosidic sweeteners or extracts from *Stevia* ssp. or *Rubus* ssp; as well as
(xi) extracts or isolated balansines from *Mycetia balansae.*

4. The substance mixture according to at least one of the claims 1 to 3, **characterized in that** they contain flavourings as optional compound (d), which are selected from the group consisting of acetophenone, allylcapronate, alpha-ionon, beta-ionon, anisaldehyde, anisylacetate, anislformate, benzaldehyde, benzothazol, bezylactetate, benzylalcohol, benzylbenzoate, butylbutyrate, butylcapronate, butylidenphtalid, carvon, camphene, caryophyllen, cineol, cinnamylacetate, citral, citronellol, citronellal, citronellylacetat, cyclohexylacetat, cymol, damascon, delta-decalactone, diacetyl, dihydrocumarin, dimethyl anthranilate, dodecalactone, acetic acid, ethylacetate, ethyl butyric acid, ethylbutyrate, ethyl caprinate, ethyl capronate, ethylcrotonate, ethylfuraneol, ethylguajakol, ethylisobutyrat, ethylisovalerianate, ethyllactate, ethyl-methylbutyrate, ethylpropionate, eucaloptol, eugenol, ethylheptylate, geraniol, geranylacetate, methyldihydrojasmonate (e.g. Hedion®), heliotropin, 2-heptanone, 3-heptanone, 4-heptanone, trans-2-heptenal, cis-4-heptenal, trans-2-hexenal, cis-3-hexenol, trans-2-hexenic acid, trans-3-hexenic acid, cis-3-hexenylacetate, cis-3-hexenylcapronate, trans-2-hexenylcapronate, cis-3-hexenyoacetate, cis-3-hexenylcapronate, cis-3-hexenylformiate, para-hydroxybenyzyl acetone, isoamylalcohol, isoamylisovalerianate, isobutylbutyrate, isobutyraldehyde, isoeugenolmethylether, isopropylmethylthiazol, lauric acid, levulinic acid, linalool, linalooloxide, linalylacetate, menthol, menthofuran, menthylanthranilate, menthylbutanol, methyl butyric acid, 2-menthylbutylacetate, menthylcapronate, menthylcinnamate, 5-methylfurfural, 3,2,2-menthylbutylacetate, menthylcapronate, mentylcinnamate, 5-methylfurfural, 3,2,2-methylcyclopentenolon, 6,5,2-mentylheptenon, methyljasmonate, 2-mentylmethylbutyrate, 2-menthyl-2-pentenolic acid, methylthiobutyrate, 3,1-methylthiohexanol, 3-methylthiohexylacetate, nerol, nerylacetate, trans,trans-2,4-nonadienal, 2,4-nonadienol, 2,6-nonadienol, nootkaton, delta octalactone, gamma octalactone, 2-octanol, 3-octanol, 1,3-octenol, 1-octylacetate, 3-octylacetate, palmitic acid, paraldehyde, phellandren, pentandion, phenylethylacetate, phenylethylalcohol, phenylethylisovalerianate, propionaldehyde, propylbutyrate, pulegon, pulegol, sinensal, sulfurol, terpinen, terpineol, terpinolen, 8,3-thiomethanone, 4,4,2-thiomethylpentanone, thymol, delta-undecalactone, gamma-undecalactone, valcen, valerianic acid, vanillin, acetoin, ethylvanillin, ethylvannilisobutyrat (=3-ethoxy-4-isobutyryloxybenzaldehyde), 2,5-dimethyl-4-hydroxy-3(2H)-furanone and derivatives thereof (preferably homofuraneol (=2-ethyl-4-hydroxy-5-methyl-3(2H)-furanone), homofuronole (=2-ethyl-5-methyl-4-hydroxy-3(2H)-fu ranone and 5-ehyl-2-methyl-4-hydroxy-3(2H)-furanone), maltol and maltol-derivates (preferably ethylmaltol), cumarin and cumarin derivatives, gamma-lactones (preferably gamma-undecalactone, gamma-nonalactone, gamma-decalactone), delta-lactones (preferably 4-methyldeltadecalactone, massoilactone, deltadecalactone, tuberolactone), methylsorbate, divanillin, 4-hydroxy-2(or 5)-ethyl-5(or 2)-methyl-3(2H)-furanone, acetic acid amylester, butyric acid isomaylester, 3-methyl-butyric acid ethylester, ethyl-3-methyl-3-phenylglycidate, ethyl-2-trans-4-cis-decadienoate, 1,1-dimethoxy-2,2,5-trimethyl-4-hexane, phenylacetaldehyde, 2-methyl-3-(methylthio)furan, 2-methyl-3-furanthiol, bis(2-methyl-3-furyl)disulphide, furfurylmercaptane, methional, 2-Acetyl-2-thiazoline, bis (2-methyl-3-furyl)disulphide, furfurylmercaptane, methionale, 2-acetyl-2-thiazoline, 3-mercapto-2-pentanone, 2,5-dimethyl-3-furanthil, 2,4,5-trimethylthiazol, 2-acetylthiazol, 2,4-dimethyl-5-ethylthiazol, 2-acetyl-1-pyrollin, 2-methyl-3-ethylpyrazin, 3-isopropyl-2-methoxypyrazin, 3-isobutyl-2-menthoxypyrazin, 2-acetylpyrazin, 2-pentylpyridin, (E,E)-2,4-decadienale, (E,E)-2,4-nonadienal, (E)-2-octenal, (E)-2-nonenal, 2-undecenal, 12-mentyltridecanal, 1-penten-3-on, guajakol, 3-hydroxy-4,5-dimethyl-2(5H)-furanone, 3-hydroxy-4-methyl-5-ethyl-2(5H)-furanone, cinammic aldehyde, cinnamic alcohol, methylsalicyclat, isopulegol as wells as (not explicitly mentioned) stereoisomers, enantiomers, positional isomers, diastereomeres, cis/trans-isomers or epimeres of these substances.

5. The substance mixtures according to any one of the claims 1 to 4, **characterized in that** they contain flavourings as optional compound (d), which are selected from the group consisting of alpha-ionon, beta-ionon, benzaldehyde, citral, damascone, delta-decalactone, diacetyl, dimethylanthranilate, dodecalactone, acetic acid, ethyl acetate, ethyl butyrate, ethyl furaneol, ethyl isobutyrate, ethyl isovalerianate, ethyl methylbutyrate, ethylpropionate, geraniol, geranylacetate, heliotropin, trans-2-hexenal, cis-3-hexenol. Cis-3-hexenylacetate, para-hydroxybenzylacetone, isobutyraldehyde, linalool, Linalylacetate, 3,2,2-methylcyclopentenolon, 2-methylmethylbutyrate, nerol, nerylacetate, vanillin, ethylvanillin, 2,5-dimethyl-4-hydroxy-3(2H)-furanone, homofuronol (=2-ethyl-5-methyl-4-hydroxy-3(2H)-furanone and 5-ethyl-2-methyl-4-hydroxy-3(2H)-furanone), maltol and maltol derivatives (preferably ethylmaltol), gamma-lactone (preferably 4-methyldeltadecalactone, massoilactone, deltadecalactone, tuberolactone), 4-hydroxy-2(or 5)-ethyl-5(or 2)-methyl-3(2H)furanone, 2-hydroxy-3-methyl-2-cyclopentenone,3-hydroxy-4,5-dimethyl-2(5H)-furanone, acetic acid isoamylester, butyric acid isoamylester, 3-methyl-butyric acid ethylester, ethyl-2-trans-4-cis-decadienoate, (E,E)-2,4-decadienal, guajakol, cinnamic aldehyde as well as (not explicitly mentioned) stereoisomers, enantiomers, positional isomers, diastereomeres, cis/trans-isomers or epimers of these substances.

6. The substance mixtures according to any one of the claims 1 to 4, **characterized in that** they contain flavourings as optional compound (d), which are selected from the group consisting of adenosine-5'-monophosphate, cytodine-5'-monophosphate, in-osin-5'-monophosphate, and their pharmaceutically acceptable salts; lactisolen; 2,4-dihydroxynezoic acid; 3-hydroxybenzoic acid; sodium salts preferably sodium chloride, sodium lactate, sodium citrate sodium acetate, sodium gluconoate; hydroxyflavanones, such as for example eriodictyol, homoeriodictyol, and their sodium salts; hydroxybenzoic acid amides, such as for example 2,4-dihydroxybenzoic acid vanillylamide, 2,4-Di-hydroxybenzoic acid-N-(4-hydroxy-3-methoxybezyl)amide, 2,4,6-tri-hydroxy-benzoic acid-N-(4-hydroxy-3-methoxybenzyl)amide, 2-hydroxy-benzoic acid-N-4-(hydroxyl-3-methoxybenzyl)amide, 4-hydroxybenzoic acid-N-(4-hydroxy-3-methoxybenzyl)-amide, 2,4-dihydroxy-benzoic acid-N-(4-hydroxy-3-methoxybenzyl)amide-mono-sodium salt, 2,4-dihydroxybenzoic acid-N-(4-hydroxy-3-ethoxybenzyl)amide, 2,4-dihydroxybenzoic acid-N-(3,4-dihydroxybenzyl)amide and 2-hydroxy-5-methoxy-N-[2-(4-hydroxy-3-methoxyphenyl)ethyl]amide and 2-hydroxy-5-methoxy-N-[2-(4-hydroxy-3-methoxyphenyl)ethyl]amide; 4-hydroxy-benzoic acid vanillylamide; hydroxydeoxybenzoinen, such as for example 2-(4-hydroxy-3-methoxyphenyl)-1-(2,4,6-trihydroxyphenyl)ethanone, 1-(2,4-dihydroxypheyl)-2-(4-hydroxy-3-methoxyphenyl)ethanone, 1-(2-hydroxy-4-methoxyphenyl)-2-(4-hydroxy-3-methoxyphenyl)ethanon); hydroxyphenylalkanones, such as for example gingerdione-[2], gingerdione-[3], gingerdione-[4], dehydrogingerdione-[2], dehydrogingerdione-[3], dehydrogingerdione-[4]); diacetyltrimers; γ-amino butyric acid and divanillins; bicyclo[4.1.0]heptan-7-carbonic acid amides; cyclopropancarbonic acid (3-methyl-cyclohexyl)amides, aromatic *Neo*-menthyl-amides; geranylamine derivatives of oxalic acid as well as neomenthyl derivatives and mixtures thereof.

7. The substance mixtures according to any one of the claims 1 to 6, **characterized in that** they contain
(a) 1 to 25 % by weight phloretin,
(b) 1 to 25 % by weight naringenin, and
(c) 1 to 50 % by weight of at least one further sweet substance, which is different from phloretin and naringenin,
with the proviso that the indications of quantity, optionally with the addition of flavourings, which form the optional component (d), summarize to 100 % by weight.

8. The substance mixtures according to at least one of the claims 1 to 7, **characterized in that** they contain with regard to the components (a) and (b) alone
(a) 5 to 99.8 % by weight phloretin,
(b1) 0.1 to 94.9 % by weight S-naringenin, and
(b2) 0.1 to 94.9 % by weight R-naringenin, wherein
in the proviso that one of the naringenin-enantiomers can be present with regard to the total content of naringenin in a weight ratio from 100:1 to 1:100 and that the indication of quantities summarizes to 100 % by weight.

9. The substance mixtures according to at least one of the claims 1 to 8, **characterized in that** they contain with regard to the componets (a) and (b) alone
(a) 5 to 90 % by weight phloretin,
(b1) 10 to 85 % by weight S-naringenin, and
(b2) 0.1 to 85 % by weight R-narringenin, wherein,
with the proviso that the indications of quantities summarize to 100 weight-%.

10. The substance mixtures according to at least one of the claims 1 to 9, **characterized in that** they contain the components (a+b) and (c) in a weight ratio of 20:80 to 80:20.

11. Oral preparations, comprising the substance mixtures according to at least one of the claims 1 to 10.

12. The preparations according to claim 11, **characterized in that** they contain the substance mixtures in quantities of 0.00001 to 2% by weight.

13. Use of naringenin to improve the taste quality of phloretin.

## Revendications

1. Mélanges de substances, contenant
(a) de la phlorétine,
(b) de la naringénine,
(c) au moins une substance édulcorante supplémentaire qui est différente de la phlorétine et de la naringénine, ainsi qu'éventuellement
(d) au moins un arôme,
les composants (a) et (b) étant contenus dans un rapport pondéral de 99:1 à 1:99.

2. Mélanges de substances selon la revendication 1, **caractérisés en ce que** les substances édulcorantes qui constituent le composant (c) sont choisies dans le groupe qui est formé par le saccharose, le tréhalose, le lactose, le maltose, le mélicitose, le raffinose, le palatinose, le lactulose, le D-fructose, le D-glucose, le D-galactose, le L-rhamnose, le D-sorbose, le D-mannose, le D-tagatose, le D-arabinose, le L-arabinose, le D-ribose, le D-glycéraldéhyde, les maltodextrines à divers degrés de polymérisation ainsi que les préparations végétales qui contiennent ces substances.

3. Mélanges de substances selon la revendication 1 et/ou la revendication 2, **caractérisés en ce que** les substances édulcorantes qui constituent le composant (c) sont choisies dans au moins l'un des groupes suivants :
(i) amidon produit par voie enzymatique ou hydrolysats de glucides tels que le sucre inverti ou le sirop de fructose ;
(ii) concentrés de fruits ;
(iii) alcools glucidiques tels que l'érythritol, le thréitol, l'arabitol, le ribotol, le xylitol, le sorbitol, le mannitol, le dulcitol ou le lactitol ;
(iv) protéines et peptides à saveur sucrée, comme la miraculine, la monelline, la thaumatine, la curculine ou la brazzéine ;
(v) édulcorants synthétiques tels que le magap, le cyclamate de sodium, l'acésulfame K, la néohespéridinedihydrochalcone, le sel de sodium de saccharine, l'aspartame, le superaspartame, le néotame, l'alitame, le sucralose, le stévioside, le rebaudioside, le rubusoside, le suavioside, le mogroside, le lugduname, le carrélame, le sucrononate, le sucrooctate, la monatine, la phénylodulcine, la phyllodulcine, ou l'abrusoside ;
(vi) édulcorants existant dans la nature, tels le stévioside, le stéviolbioside, le rébaudioside A, d'autres stéviolglycosides tels que le rébaudioside B, le rébaudioside C, le rébaudioside D, lé rébaudioside E, le rébaudioside F, le rébaudioside G, le rébaudioside H, le dulcoside et/ou le rubusoside, l'oslandine, le polypodoside A, la strogine 1, la strogine 2, la strogine 4, la sélligueanine A, le dihydroquercétine-3-acétate, la périllartine, le télosmoside A15, la périandrine I-V, la phyllodulcine, les ptérocaryosides, les cyclocaryosides, les mukuroziosides, le trans-anéthol, le trans-cinnamaldéhyde, les bryosides, les bryonosides, les bryonodulcosides, les carnosiflosides, les scandénosides, les gypénosides, la trilobatine, la phloridzine, les dihydroflavanols, l'hématoxyline, la cyanine, l'acide chlorogénique, l'albiziasaponine, les télosmosides, le gaudichaudioside, les mogrosides, l'hernandulcine, l'acide glycyrrhétinique, la balansine A, la balansine B ;
(vii) extraits ou fractions enrichies de ces extraits, comme par exemple extraits de Thaumatococcus (katemfe), extraits de *Stevia* ssp. (en particulier *Stevia rebaudiana*), extrait de swingle (*Momordica* ou. *Siratia grosvenorii,* Luo-Han-Guo), extraits de racine de réglisse, également *Glycerrhyzia* ssp. (en particulier *Glycerrhyzia glabra*), *Rubus* ssp. (en particulier *Rubus suavissimus*), extraits d'agrumes, extraits d'*Hydrangea macrophylla* var. *thunbergii* Makino, extraits de *Lippia dulcis,* extraits de *Mycetia balansae ;*
(viii) acides aminés à saveur sucrée (par exemple glycine, D-leucine, D-thréonine, D-asparagine, D-phénylalanine, D-tryptophane, L-proline) ;
(ix) substances de faible masse moléculaire à saveur sucrée, telles que l'hernandulcine, les dihydrochalconeglycosides, la glycyrrhizine, l'acide glycyrrhétinique, leurs dérivés et sels, extraits de réglisse (*Glycyrrhizza glabra* ssp.), extraits de *Lippia dulcis,* extraits de *Momordica* ssp. ou préparations à base de fruits ou extraits par exemple de *Momordica grosvenori* [Luo Han Guo], *Hydrangea dulcis, Stevia* ssp. (par exemple *Stevia rebaudiana*) ;
(x) édulcorants glycosidiques traités par voie enzymatique ou extraits de *Stevia* ssp. ou *Rubus* ssp ; ainsi que
(xi) extraits ou balansines isolées de *Mycetia balansae.*

4. Mélanges de substances selon au moins l'une quelconque des revendications 1 à 3, **caractérisés en ce qu'**ils contiennent en tant que composants (d) optionnels des arômes qui sont choisis dans le groupe qui est formé par l'acétophénone, le caproate d'allyle, l'alphalonone, la bêtalonone, l'anisaldéhyde, l'acétate d'anisyle, le formiate d'anisyle, le benzaldéhyde, le benzothiazol, l'acétate de benzyle, l'alcool benzylique, le benzoate de benzyle, le butyrate de butyle, le caproate de butyle, le butylidènephtalide, la carvone, le camphène, le caryophyllène, le cinéol, l'acétate de cinnamyle, le citral, le citronellol, le citronellal, l'acétate de citronellyle, l'acétate de cyclohexyle, le cymol, la damascone, la delta-décalactone, le diacétyle, la dihydrocoumarine, l'anthranilate de diméthyle, la dodécalactone, l'acide acétique, l'acétate d'éthyle, l'acétate d'éthoxyéthyle, l'acide éthylbutyrique, le butyrate d'éthyle, le caprate d'éthyle, le caproate d'éthyle, le crotonate d'éthyle, l'éthylfuranéol, l'éthyl-guaiacol, l'isobutyrate d'éthyle, l'isovalérate d'éthyle, le lactate d'éthyle, le butyrate d'éthylméthyle, le propionate d'éthyle, l'eucalyptol, l'eugénol, l'heptylate d'éthyle, le géraniol, l'acétate de géranyle, le dihydrojasmonate de méthyle (par exemple Hedion®), l'héliotropine, la 2-heptanone, la 3-heptanone, la 4-heptanone, le trans-2-hepténal, le cis-4-hepténal, le trans-2-hexénal, le cis-3-hexénol, l'acide trans-2-hexénoïque, l'acide trans-3-hexénoïque, l'acétate de cis-3-hexényle, le caproate de cis-3-hexényle, le caproate de trans-2-hexényle, le formiate de cis-3-hexényle, la para-hydroxybenzylacétone, l'alcool isoamylique, le valérate d'isoamyle, le butyrate d'isobutyle, l'isobutyraldéhyde, l'éther isoeugénolméthylique, l'isopropylméthylthiazole, l'acide laurique, l'acide lévulinique, le linalool, l'oxyde de linalool, l'acétate de linalyle, le menthol, le menthofurane, l'anthranilate de méthyle, le méthylbutanol, l'acide méthylbutyrique, l'acétate de 2-méthylbutyle, le caproate de méthyle, le cinnamate de méthyle, le 5-méthylfurfural, la 3,2,2-méthylcyclopenténolone, la 6,5,2-méthylhepténone, le jasmonate de méthyle, le butyrate de 2-méthylméthyle, l'acide 2-méthyl-2-penténoïque, le thiobutyrate de méthyle, le 3,1-méthylthiohexanol, l'acétate de 3-méthylthiohexyle, le nérol, l'acétate de néryle, le trans,trans-2,4-nonadiénal, le 2,4-nonadiénol, le 2,6-nonadiénol, la nootkatone, la delta-octalactone, la gamma-octalactone, le 2-octanol, le 3-octanol, le 1,3-octénol, l'acétate de 1-octyle, l'acétate de 3-octyle, l'acide palmitique, le paraldéhyde, le phéllandrène, la pentandione, l'acétate de phényléthyle, l'alcool phenyléthylique, l'isovalérate de phényléthyle, le propionaldéhyde, le butyrate de propyle, la pulégone, le pulégol, le sinensal, le sulfurol, le terpinène, le terpinéol, les terpinols, la 8,3-thiomenthanone, la 4,4,2-thiométhylpentanone, le thymol, la delta-undécalactone, la gamma-undécalactone, le valencène, l'acide valérique, la vanilline, l'acétoïne, l'éthylvanilline, l'isobutyrate d'éthylvanilline (= 3-éthoxy-4-isobutyryloxybenzaldéhyde), la 2,5-diméthyl-4-hydroxy-3(2H)-furanone et ses dérivés (dont de préférence l'homofuranéol (= 2-éthyl-4-hydroxy-5-méthyl-3(2H)-furanone), l'homofuronol (= 2-éthyl-5-méthyl-4-hydroxy-3(2H)-furanone et la 5-éthyl-2méthyl-4-hydroxy-3(2H)-furanone), le maltol et les dérivés de maltol (dont de préférence l'éthylmaltol), la coumarine et les dérivés de coumarine, la gamma-lactone (dont de préférence la gamma-undécalactone, la gamma-nonalactone, la gamma-décalactone), la delta-lactone (dont de préférence la 4-méthyldeltadécalactone, la massoilactone, la delta-décalactone, la tubérolactone), le sorbate de méthyle, la divanilline, la 4-hydroxy-2(ou 5)-éthyl-5(ou 2)-méthyl-3(2H)furanone, la 2-hydroxy-3-méthyl-2-cyclopenténone, la 3-hydroxy-4,5-di-méthyl-2(5H)-furanone, l'acétate d'isoamyle, le butyrate d'isoamyle, le 3-méthyl-butyrate d'éthyle, le glycidate d'éthyl-3-méthyl3-phényle, le 2-trans-4-cis-décadiénoate d'éthyle, le 1,1-diméthoxy-2,2,5-triméthyl-4-hexane, le phénylacétaldéhyde, le 2-méthyl-3-(méthylthio)furane, le 2-méthyl-3-furanethiol, le disulfure de bis(2-méthyl-3-furyle), le furfurylmercaptan, le méthional, la 2-acétyl-2-thiazoline, la 3-mercapto-2-pentanone, le 2,5-diméthyl-3-furanethiol, le 2,4,5-triméthylthiazole, le 2-acétylthiazole, le 2,4-diméthyl-5-éthylthiazole, la 2-acétyl-1-pyrroline, la 2-méthyl-3-éthylpyrazine, la 2-éthyl-3,5-diméthylpyrazine, la 2-éthyl-3,6-diméthylpyrazine, la 2,3-diéthyl-5-méthylpyrazine, la 3-isopropyl-2-méthoxypyrazine, la 3-isobutyl-2méthoxypyrazine, la 2-acétylpyrazine, 2-pentylpyridine, le (E,E)-2,4-décadiénal, le (E,E)-2,4-nonadiénal, le (E)-2-octénal, le (E)-2-nonénal, le 2-undécénal, le 12-méthyltridécanal, la 1-pentén-3-one, le gaïacol, la 3-hydroxy- 4,5-diméthyl-2(5H)-furanone, la 3-hydroxy-4-méthyl-5-éthyl-2(5H)-furanone, le cinnamaldéhyde, l'alcool cinnamique, le salicylate de méthyle, l'isopulégol ainsi que (non nommés explicitement ici) les stéréoisomères, énantiomères, isomères de position, diastéréoisomères, isomères cis/trans ou épimères de ces substances.

5. Mélanges de substances selon au moins l'une quelconque des revendications 1 à 4, **caractérisés en ce qu'**ils contiennent en tant que composants (d) optionnels des arômes qui sont choisis dans le groupe qui est formé par l'alpha-lonone, la bêtalonone, le benzaldéhyde, le citral, la damascone, la delta-décalactone, le diacétyle, l'anthralinate de diméthyle, la dodécalactone l'acide acétique, l'acétate d'éthyle, le butyrate d'éthyle, l'éthylfuranéol, l'isobutyrate d'éthyle, l'isovalérate d'éthyle, le butyrate d'éthylméthyle, le propionate d'éthyle, le géraniol, l'acétate de géranyle, l'héliotropine, le trans-2-hexénal, le cis-3-hexénol, l'acétate de cis-3-hexényle, la para-hydroxybenzylacétone, l'isobutyraldéhyde, le linalool, l'acétate de linalyle, la 3,2,2-méthylcyclopentenolone, le butyrate de 2-méthylméthyle, le nérol, l'acétate de néryle, la vanilline, l'éthylvanilline, la 2,5-diméthyl-4-hydroxy- 3(2H)-furanone, l'homofuronol (= 2-éthyl-5-méthyl-4-hydroxy-3(2H)-furanone et la 5-éthyl-2-méthyl-4-hydroxy-3(2H)-furanone), le maltol et les dérivés de maltol (dont de préférence l'éthyl-maltol), les gamma-lactones (dont de préférence la gamma-undécalactone, la gamma-nonalactone, la gamma-décalactone), la delta-lactone (dont de préférence la 4-méthyldeltadécalacton, la massoilactone, la delta-décalactone, la tubérolactone), la 4-hydroxy-2(ou 5)-éthyl-5(ou 2)méthyl-3(2H)furanone, la 2-hydroxy-3-méthyl-2-cyclopenténone, la 3-hydroxy4,5-diméthyl-2(5H)-furanone, l'acétate d'isoamyle, le butyrate d'isoamyle, le 3-méthyl-butyrate d'éthyle, le 2-trans-4-cis-décadiénoate d'éthyle, le (E,E)-2,4-décadiénal, le guaïacol, le cinnamaldéhyde ainsi que (non nommés explicitement ici) les stéréoisomères, énantiomères, isomères de position, diastéréoisomères, isomères cis/trans ou épimères de ces substances.

6. Mélanges de substances selon au moins l'une quelconque des revendications 1 à 4, **caractérisés en ce qu'**ils contiennent en tant que composants (d) optionnels des arômes qui sont choisis dans le groupe qui est formé par l'adénosine-5'-monophosphate, le cytidine-5'-monophosphate, l'inosine-5'monophosphate, et leurs sels pharmaceutiquement acceptables ; les lactisols ; l'acide 2,4-dihydroxybenzoïque ; l'acide 3-hydroxybenzoïque ; les sels de sodium, de préférence le chlorure de sodium, le lactate de sodium, le citrate de sodium, l'acétate de sodium, le gluconate de sodium ; les hydroxyflavanones, comme par exemple l'ériodictyol, l'homoériodictyol, et leurs sels de sodium ; les amides d'acide hydroxybenzoïque, comme par exemple le vanillylamide d'acide 2,4-dihydroxybenzoïque, le *N*-(4-hydroxy-3-méthoxybenzyl)amide d'acide 2,4-dihydroxybenzoïque, le *N*-(4-hydroxy-3-méthoxybenzyl)amide d'acide 2,4,6-trihydroxybenzoïque, le *N*-(4-(hydroxy-3-méthoxybenzyl)amide d'acide 2-hydroxybenzoïque, le *N*-(4-hydroxy-3-méthoxybenzyl)amide d'acide 4-hydroxybenzoïque, le sel monosodique de *N*-(4-hydroxy-3méthoxybenzyl)amide d'acide 2,4-dihydroxybenzoîque, le *N*-(2-(4-hydroxy-3méthoxyphényl)-éthyl-amide d'acide 2,4-dihydroxybenzoïque, le *N*-(4-hydroxy3-éthoxybenzyl)amide d'acide 2,4-dihydroxybenzoïque, le *N*-(3,4-dihydroxybenzyl)amide d'acide 2,4-dihydroxybenzoïque et le 2-hydroxy-5-méthoxy-N-[2(4-hydroxy-3-méthoxyphényl)éthyl]amide ; le vanillylamide d'acide 4-hydroxybenzoïque ; les hydroxydésoxybenzoïnes, comme par exemple la 2-(4-hydroxy-3-méthoxyphényl)-1-(2,4,6-trihydroxyphényl)éthanone, la 1-(2,4dihydroxyphényl)-2-(4-hydroxy-3-méthoxyphényl)éthanone, la 1-(2-hydroxy4-méthoxyphényl)-2-(4-hydroxy-3-méthoxyphényl)éthanone) ; les hydroxyphénylalcanediones, comme par exemple la gingerdione-[2], la gingerdione-[3], la gingerdione-[4], la déhyrogingerdione-[2], la déhydrogingerdione-[3], la déhydrogingerdione-[4]) ; les trimères de diacétyle ; les acides γ-aminobutyriques et divanillines ; les bicyclo[4.1.0]heptan-7-carboxamides ; les (3-méthyl-cyclohexyl)amides d'acide cyclopropanecarboxylique, les néo-menthylamides aromatiques ; les dérivés géranylamide d'acide oxalique ainsi que les dérivés néomenthyle et leurs mélanges.

7. Mélanges de substances selon au moins l'une quelconque des revendications 1 à 6, **caractérisés en ce qu'**ils contiennent
(a) 1 à 25 % en poids de phlorétine,
(b) 1 à 25 % en poids de naringénine, et
(c) 1 à 50 % en poids d'au moins une substance édulcorante supplémentaire, qui est différente de la phlorétine et de la naringénine
étant entendu que la somme des données quantitatives, éventuellement avec addition d'arômes qui constituent le composant optionnel (d), est égale à 100 % en poids.

8. Mélanges de substances selon au moins l'une quelconque des revendications 1 à 7, **caractérisés en ce qu'**ils contiennent, par rapport aux composants (a) et (b) seuls
(a) 5 à 99,8 % en poids de phlorétine,
(b1) 0,1 à 94,9 % en poids de S-naringénine, et
(b2) 0,1 à 94,9 % en poids de R-naringénine,
étant entendu qu'eu égard à la quantité totale de naringénine, l'un des énantiomères de naringénine peut être présent dans un rapport pondéral de 100 :1 à 1 :100 et que la somme des données quantitatives est égale à 100 % en poids.

9. Mélanges de substances selon au moins l'une quelconque des revendications 1 à 8, **caractérisés en ce qu'**ils contiennent, par rapport aux composants (a) et (b) seuls
(a) 5 à 90 % en poids de phlorétine,
(b1) 10 à 85 % en poids de S-naringénine, et
(b2) 0,1 à 85 % en poids de R-naringénine,
étant entendu que la somme des données quantitatives est égale à 100 % en poids.

10. Mélanges de substances selon au moins l'une quelconque des revendications 1 à 9, **caractérisés en ce qu'**ils contiennent les composants (a+b) et (c) dans un rapport pondéral de 20:80 à 80:20.

11. Préparations orales, contenant les mélanges de substances selon au moins l'une quelconque des revendications 1 à 10.

12. Préparations selon la revendication 11, **caractérisées en ce qu'**elles contiennent les mélanges de substances en des quantités de 0,00001 à 2 % en poids.

13. Utilisation de la naringénine pour l'amélioration de la qualité gustative de la phlorétine.
